# EUROPEAN PATENT APPLICATION

(11) **EP 4 327 830 A1**
(43) Date of publication of application: **28.02.2024**
(21) Application number: 22192517.5
(22) Date of filing: 27.08.2022
(51) Int. Cl.: A61K 47/68, A61P 35/00

(54) **A METHOD OF MODIFYING AN ORGANIC CYTOTOXIN FOR USE AS A PAYLOAD IN AN ANTIBODY-DRUG CONJUGATE AND MODIFIED ORGANIC CYTOTOXINS DERIVED THEREFROM**

(71) Applicant: Simris Biologics GmbH, 12489 Berlin (DE)
(72) Inventor: ENKE, Heike, 12307 Berlin (DE); ENKE, Dan, 12307 Berlin (DE); NIEDERMEYER, Timo, 06114 Halle (Saale) (DE); STOCK, Laura, 06112 Halle (Saale) (DE); SCHUSTER, Sabine, 06120 Halle (Saale) (DE)
(74) Representative: Andresen, Heiko

(57) **Abstract**

This invention provides a method of modifying a cyanobacterial or fungal toxin for use as a payload in an antibody-drug conjugate, comprising the following steps: selecting a cyanobacterial or fungal toxin having a base structure known to be cytotoxic for a target cell after uptake by a transporter protein of the target cell, modifying the base structure with at least one structural modification, wherein the at least one structural modification comprises an organic inhibitory group or effector molecule reducing the uptake of the modified toxin by the transporter protein into the target cell in comparison to the base structure, and an organic coupling group configured for covalent conjugation of the modified toxin to an antibody. This invention further provides modified toxins derived from the method and uses thereof.

## Description

### Sequence Listing

This application contains an electronic sequence listing in xml-format according to WIPO ST.26 standard as part of the description, the content of which is hereby incorporated by reference in its entirety.

### Field of invention

This invention relates to biologically active cytotoxic anticancer payloads or drugs. In particular, this invention relates to modified organic cytotoxins, more particularly modified cyanobacterial and fungal toxins, for use as a payload in an antibody-drug conjugate. This invention further relates to antibody-drug conjugates comprising such biologically active cytotoxic anticancer payloads coupled to an antibody that specifically targets a certain tumor antigen.

### Background of invention

Today, cancer is the second leading cause of death globally, accounting for about 10 million deaths per year worldwide. Due to global population growth and aging, cancer incidence rates have increased over the past decades and are expected to continue to rise.

Most anticancer drugs currently used in conventional chemotherapy have the disadvantage that they are generally cytotoxic, as they insufficiently discriminate between cancerous cells and healthy tissues, with known severe side effects and significant limitations on the quality of life of patients.

The need for anticancer drugs with a specific spectrum of action against malignant cells has driven the development of antibody-drug conjugates.

Antibody-drug conjugates, in the following also referred to as "ADCs", are a class of biopharmaceutical drugs designed as a targeted therapy for treating cancer. Unlike chemotherapy, ADCs are intended to target and kill tumor cells while sparing healthy cells. ADCs are biopharmaceuticals essentially composed of a monoclonal antibody linked to a biologically active and often cytotoxic drug, herein also referred to as the "payload", thereby combining the target specificity of a monoclonal antibody with the cancer-killing ability of a cytotoxic drug.

Conceptually, ADCs are supposed to target tumor cells and spare healthy tissues, mediated by the target specificity of the monoclonal antibody. Nevertheless, off-target effects are often seen during clinical application, caused for example by spontaneous or enzymatic hydrolysis of the chemical linkage between drug and antibody during transport and metabolism after systemic administration, or by the release of the payload from lysing or killed cancer cells into the surrounding tissue.

Current efforts to reduce or eliminate the undesirable toxic side effects on healthy cells are mainly directed to improving the stability of the chemical bonds between the cytotoxic payload and the monoclonal antibody in order to avoid premature release of the drug outside the tumor cell.

For example, WO 2018/206715 A2 and WO 2018/219619 A1, the disclosures of which are hereby incorporated by reference in their entirety, disclose modified cyanobacterial microcystins and nodularins as cytotoxic payloads which incorporate, among others, non-naturally occurring amino acids providing a chemical anchor group for the coupling of the payload to a linker or an antibody, respectively, thereby leading to homogeneous and stably loaded ADCs with limited payload release in the blood stream.

However, an improved chemical linkage between the antibody and the cytotoxic payload can only partially overcome the above-noted drawbacks of currents ADCs. In particular, it does not solve the problem of payload release after the targeted cancer cells died. Furthermore, the high potency of the cytotoxins creates unresolved health and safety risks in their manufacture and handling, resulting in cost-intensive infrastructure and production.

Against this background, it is an object of the present invention to provide improved cytotoxins for use as a payload in an antibody-drug conjugate. Another object of the present invention is to provide an improved antibody-drug conjugate.

These objectives are solved in accordance with the subject matters of the independent claims. Preferred embodiments are subject matters of the dependent claims and the following description.

### Summary of invention

In certain embodiments, the invention provides a method of modifying a cyanobacterial or fungal toxin for use as a payload in an antibody-drug conjugate, comprising the following steps: selecting a cyanobacterial or fungal toxin having a base structure known to be cytotoxic for a target cell after uptake by a transporter protein of the target cell; modifying the base structure with at least one structural modification, wherein the at least one structural modification comprises an organic inhibitory group or effector molecule reducing the uptake of the modified toxin by the transporter protein into the target cell in comparison to the base structure, and an organic coupling group configured for covalent conjugation of the modified toxin to an antibody.

In certain embodiments, the invention provides a method of modifying a cyanobacterial or fungal toxin for use as a payload in an antibody-drug conjugate, comprising the following steps: selecting a cyanobacterial or fungal toxin having a base structure known to be cytotoxic for a target cell after uptake by a transporter protein of the target cell; modifying the base structure with (i) at least an organic inhibitory group or effector molecule reducing the uptake of the modified toxin by the transporter protein into the target cell in comparison to the base structure, and (ii) an organic coupling group configured for covalent conjugation of the modified toxin to an antibody.

In certain embodiments, the invention provides a method for manufacturing a modified cyanobacterial or fungal toxin, comprising the following steps: A) providing a toxin having a base structure known to be cytotoxic for a target cell after uptake by a transporter protein of the target cell and, B) introducing at least one structural modification into the toxin, wherein the at least one structural modification comprises (i) an organic inhibitory group or effector molecule reducing the uptake of the modified toxin by the transporter protein into the target cell in comparison to the base structure, and (ii) an organic coupling group configured for covalent conjugation of the modified toxin to an antibody.

In certain embodiments, the invention provides a method for manufacturing a modified cyanobacterial or fungal toxin, comprising the following steps: A) providing a toxin having (i) a base structure known to be cytotoxic for a target cell after uptake by a transporter protein of the target cell and (ii) at least one functional group configured for and/or capable of covalent conjugation, B) introducing at least one structural modification into the toxin via covalent conjugation to the functional group of the toxin, wherein the at least one structural modification comprises (i) an organic inhibitory group or effector molecule reducing the uptake of the modified toxin by the transporter protein into the target cell in comparison to the base structure, and (ii) an organic coupling group configured for covalent conjugation of the modified toxin to an antibody.

In certain embodiments, the invention provides a modified cyanobacterial or fungal toxin, comprising a base structure having a cytotoxicity for a target cell after uptake by a transporter protein of the target cell and at least one structural modification of the base structure, wherein the at least one structural modification comprises an organic inhibitory group or effector molecule reducing the uptake of the modified toxin by the transporter protein into the target cell in comparison to the base structure, and an organic coupling group configured for covalent conjugation of the organic cytotoxin to an antibody.

In certain embodiments, the invention provides use of a modified toxin as defined above in manufacturing an antibody-drug conjugate, comprising covalently conjugating the modified toxin via the organic coupling group to an antibody or a fragment thereof comprising an antigen-specific binding site.

In certain embodiments, the invention provides a method for manufacturing an antibody-drug conjugate, comprising the following steps: a) providing a monoclonal antibody or a fragment thereof comprising an antigen-specific binding site and a toxin as defined above, b) covalently conjugating the toxin to the antibody via the organic coupling group.

In certain embodiments, the invention provides a method for manufacturing an antibody-drug conjugate, comprising the following steps: a) providing a toxin having (i) a base structure known to be cytotoxic for a target cell after uptake by a transporter protein of the target cell, and (ii) at least a first functional group and a second functional group configured for, or capable of, covalent conjugation, b) introducing at least one structural modification into the toxin via covalent conjugation to the first functional group, wherein the at least one structural modification comprises an organic inhibitory group or effector molecule reducing the uptake of the modified toxin by the transporter protein into the target cell in comparison to the base structure, and c) covalently conjugating the toxin to an antibody via the second functional group, thereby manufacturing the antibody-drug conjugate.

In certain embodiments, the invention provides an antibody-drug conjugate comprising a modified toxin as defined above and an antibody or a fragment thereof comprising an antigen-specific binding site, wherein the antibody or the fragment thereof is attached to the modified toxin via the organic coupling group.

In certain embodiments, the invention provides a modified cyanobacterial or fungal toxin or an antibody-drug conjugate as defined above for use in the treatment of a malignant disease.

In certain embodiments, the invention provides a method for treating a human subject afflicted with a malignant disease, comprising administering to the subject a therapeutically effective dosing regimen of the antibody-drug conjugate defined above.

In some embodiments according to, or as applied to, any of the embodiments above, the base structure comprises an oligopeptide, in particular a cyclic oligopeptide, more particularly a cyclic oligopeptide containing at least one non-proteinogenic amino acid.

In some embodiments according to, or as applied to, any of the embodiments above, the modified toxin or the base structure, respectively, is selected from a group consisting of a microcystin, a nodularin or an amatoxin such as an amanitin. In particular embodiments, the modified toxin or the base structure, respectively, is a microcystin.

In some embodiments according to, or as applied to, any of the embodiments above, the base structure is a naturally occurring toxin.

In some embodiments according to, or as applied to, any of the embodiments above, the base structure is non-naturally occurring, in particular a base structure of a naturally occurring toxin that has been modified with at least one functional group, preferably one or two functional groups, configured for, or capable of, chemical conjugation to another organic molecule, such as an antibody or a modifying molecule.

In some embodiments according to, or as applied to, any of the embodiments above, the chemical conjugation is a bioorthogonal chemical conjugation, preferably comprising a click chemistry reaction.

In some embodiments according to, or as applied to, any of the embodiments above, the organic inhibitory group is selected from a group consisting of an organic group carrying at least one charge at a physiological pH-value, a heterocycle, e. g. biotin, an organic formamide, an alcohol, or any combination thereof.

In some embodiments according to, or as applied to, any of the embodiments above, the transporter protein comprises an organic anion transporter polypeptide (OATP), in particular OATP1B1 and/or OATP1B3.

In some embodiments according to, or as applied to, any of the embodiments above, the target cell is a mammalian cell, in particular a cancer cell.

Preferred variants of these embodiments can be derived from the description of the invention and the detailed description of embodiments.

### Description of invention

A first aspect of the present invention relates to a method of modifying a cyanobacterial or fungal toxin for use as a payload in an antibody drug conjugate.

The method comprises the following steps: selecting a cyanobacterial or fungal toxin having a base structure known to be cytotoxic for a target cell after uptake by a transporter protein of the target cell; and modifying the base structure with at least one structural modification, the toxin thereby being modified. Herein, the at least one structural modification comprises an organic inhibitory group or effector molecule reducing the uptake of the modified toxin by the transporter protein into the target cell in comparison to the base structure, and an organic coupling group configured for covalent conjugation of the modified toxin to an antibody.

The invention is based on the inventors' insight that cytotoxins generally must be taken up by cells either through active transport mediated by transport proteins or by passive diffusion before they can exert their cytotoxic function, whereas when coupled to an antibody, the internalization of the cytotoxins changes to receptor-mediated endocytosis, e. g. clathrin-mediated endocytosis, caveolae-mediated endocytosis or pinocytosis, a process by which the cells absorb the ADC by the inward budding of the plasma membrane after binding of the antibody to the cell surface, thereby transporting the toxins into the targeted cells. Based on this insight, the inventors realized the potential of modifying toxins which, in an isolated form, require active transport across a cell membrane and cannot enter a cell by passive diffusion, with a structural modification that intentionally impairs their acceptance by the corresponding transporter proteins, so that they contain a structural "safety catch" that prevents them from being actively taken up into the cell and exerting their cytotoxic function. In this way, the modified toxins according to the present invention are blocked from unfolding unspecific cytotoxic activity as isolated compounds in an extracellular environment in the human body, e. g. upon premature release from the ADC into the bloodstream or after release from a dead cancer cell. In contrast, when the modified toxins of the present invention are bound to an antibody via the provided organic coupling group, they are internalized by target cells as the antibody's payload through receptor-mediated endocytosis where, after intracellular trafficking and processing of the ADC, the toxin is unloaded and the cytotoxicity of the toxin is unleashed to trigger tumor cell death. In this way, the present invention provides modified toxins for use as ADC payloads with significantly enhanced safety profile, which allows to increase the dosage of the ADC for improved therapeutic benefit without resulting in unacceptable side-effects or toxicity. In addition, safety during production and processing of the modified toxins is improved.

Toxins with a base structure that requires a transporter protein for cell uptake of the toxin are known to the skilled artisan. For example, transport of the toxins or the base structure, respectively, can be mediated by an organic anion transporting polypeptide (OATP) or subtype thereof, such as OATP1B1, OATP1B3 and/or OATP1A2. These OATPs are primarily found in the liver of humans. Therefore, the inventors have realized the importance of blocking transport of payload toxins by OATPs to circumvent hepatotoxicity and improve the relative safety of the ADC.

In general, the base structure can be a naturally occurring toxin or a base structure thereof. The base structure can also be non-naturally occurring, such as a base structure of a naturally occurring toxin that has been modified with at least one anchor group configured for chemical conjugation to an antibody, as described for example in WO 2018/206715 A2.

Preferably, the toxin is an oligopeptide toxin, i. e. the base structure comprises an oligopeptide with several amino acids, e. g. 5 to 15 or 5 to 10 amino acids, in particular five, six, seven or eight amino acids. More preferably, the oligopeptide is cyclic. Preferably, the cyclic oligopeptide toxin comprises at least one non-proteinogenic amino acid.

Nonlimiting examples of suitable cyanobacterial or fungal toxins in the sense of the present invention include microcystins, nodularins and amatoxins such as amanitins.

In preferred embodiments, the cyanobacterial toxin is a microcystin. Microcystins are cyanobacterial cyclic heptapeptides that potently inhibit eukaryotic serine/threonine protein phosphatases type 1 and 2A, leading to an interruption of many essential signal transduction pathways and finally to the collapse of the cytoskeleton and the death of the cell. Microcystins are primarily taken up by OATP1B1 and OATP1B3.

In other preferred embodiments, the cyanobacterial toxin is a nodularin. Nodularins are cyanobacterial cyclic pentapeptides evolutionary related to microcystins that also inhibit protein phosphatases type 1 and 2A as their cytotoxic mode of action and that are primarily taken up by OATP1B1.

In yet other preferred embodiments, the fungal toxin is an amanitin. Amanitins are fungal bicyclic octapeptides belonging to the subgroup of amatoxins. The most prominent alpha-Amanitin is an inhibitor of RNA polymerase II, binding to the bridging helix of RNA polymerase II and inhibiting the translocation of RNA and DNA needed to empty the site for the next synthesis run.

In preferred embodiments, the method comprises incorporating the at least one structural modification in, or attaching the at least one structural modification to, a side-chain of at least one of the amino acids. As used herein, incorporating the at least one structural modification in a side-chain of an amino acid includes a case wherein the structural modification or a part thereof is synthetically or biosynthetically introduced into the base structure e. g. via amino acid building blocks or through feeding of amino acid precursors which contain the structural modification or part thereof, as described for instance in WO 2018/219619 A1. Attaching the at least one structural modification to a side-chain of an amino acid includes a case wherein the structural modification is chemically conjugated to a functional group of the side-chain capable of such chemically conjugation. The functional group can be a functional group of an amino acid naturally occurring in the base structure such as an amino group, a carboxyl group, a hydroxy group, an aldehyde group, a keto group or a thiol group. The functional group can also be a group that does not occur in a natural counterpart of the base structure, e. g. a functional group that has been artificially introduced into the base structure as described in WO 2018/219619 A1. Preferably, the functional group is configured for biorthogonal conjugation, i. e. a chemical conjugation reaction that can occur inside of living systems without interfering with native biochemical processes. Preferably, the functional group is selected from a group consisting of azido group, alkyne group, alkene group, phosphine, phosphonate, tetrazine, hydrazine, hydrazone, oxime, and any combinations thereof.

In preferred embodiments, the at least one structural modification comprises a single organic moiety that includes both the organic inhibitory group or the effector molecule, respectively, and the organic coupling group. In some embodiments hereunder, the organic inhibitory group and the organic coupling group can be the same organic group or structurally overlapping organic groups which share one or more carbon atoms and/or heteroatoms. In particular, the organic coupling group can be a part of the organic inhibitory group.

In other preferred embodiments, the at least one structural modification comprises a first structural modification comprising the organic inhibitory group or effector molecule and a second structural modification comprising the organic coupling group. Accordingly, modifying the base structure can comprise incorporating the first structural modification in, or attaching the first structural modification to, a side-chain of a first amino acid of the base structure, and incorporating the second structural modification in, or attaching the second structural modification to, a side-chain of a second amino acid of the base structure which is different from the first amino acid. In preferred embodiments hereunder, the first structural modification comprises attaching an organic molecule containing, or consisting of, the organic inhibitory group or effector molecule to the side-chain of the first amino acid of the base structure, and incorporating the second structural modification containing, or consisting of, the organic coupling group configured for covalent conjugation of the toxin to the antibody, in particular a functional group configured for biorthogonal conjugation, in the side-chain of the second amino acid.

As used herein, selecting a cyanobacterial or fungal toxin can in principle be a mental step or a step that is performed *in silico.* On the other hand, modifying the base structure with at least one structural modification can comprise substeps for manufacturing the modified toxin, such as (i) providing the base structure, and (ii) modifying the base structure with the at least one structural modification. Advantageously, the step of modifying the base structure with the at least one structural modification can be performed by semi-synthesis, also known as partial chemical synthesis, wherein the base structure is isolated from a natural source such as a cyanobacterial cell culture as described in WO 2018/219619 A1 or a fungal cell culture, respectively, and then chemically introducing the at least one structural modification to the base structure to produce the modified toxin. In this way, the invention provides unprecedented access to a wide structural variety of improved toxins for use in ADCs.

In preferred embodiments, the organic inhibitory group comprises an organic group carrying at least one charge at a physiological pH-value, i. e. a pH of 6-8 in an aqueous medium at 20-40 °C.

Preferably, the organic inhibitory group comprises an amphoteric group or a zwitterionic group, respectively, with at least one cationic charge and at least one anionic charge at a physiological pH-value. The cationic charge can be based on primary, secondary, and/or tertiary amines as well as quaternary ammonium cations and guanidine, for example. The anionic part can e. g. be based on sulfonates, sulfinates, phenolic hydroxyl groups, thiols, carboxylates and/or thiocarboxylates. Preferably, the organic amphoteric group is an amino acid group, wherein the at least one positive charge is based on an amino group and the at least one negative charge is based on a carboxylic acid group.

The inventors discovered that the aforementioned organic inhibitory groups are potent inhibitors of transporter proteins while surprisingly enhancing the cytotoxic effect of the modified toxin compared to the base structure without the structural modification. Through to this synergistically acting double effect, the modified toxins of the present invention offer an improved therapeutic window in comparison to conventional toxins and achieving greater therapeutic benefit without resulting in unacceptable side-effects or toxicity. In this regard, references is also made to the detailed description of embodiments set out below.

In further preferred embodiments, the organic inhibitory group comprises a heterocycle, in particular a five-membered or six-membered ring with at least one or at least two heteroatoms, saturated or unsaturated, optionally substituted, in particular having one to three heteroatoms from the group N, O and S. Preferably, the heterocycle contains at least one sulfur atom and/or at least one nitrogen atom in the ring. The organic inhibitory group can also comprise a ring system that comprises, or consists of, two fused heterocycles, saturated or unsaturated and optionally substituted. For example, the organic inhibitory group can be biotin.

In further preferred embodiments, the organic inhibitory group comprises an organic formamide and/or an alcohol. Suitable formamides and alcohols are exemplified in the detailed description of embodiments below.

Some embodiments provide that the at least one structural modification comprises an effector molecule which is a known inhibitor of active transporter, or a functional part or derivative of such inhibitor. In some embodiments, the effector molecule comprises a competitive or allosteric inhibitor ligand of the transporter protein or a part or derivative thereof having at least partially the competitive or allosteric inhibiting function of the ligand. In particular, the effector molecule can be a known inhibitor of an OATP, either with specific inhibitory activity against an OATP subtype or with inhibitory activity against two or more OATP subtypes. Suitable inhibitors are known to the skilled artisan including, but not limited to, pravastatin, erlotinib, cyclosporin A, rifampicin, nystatin, doxorubicin, and ritonavir, as well as flavonoids and nostocyclopeptide or a functional part or derivative thereof.

As noted above, the organic coupling group is a functional group, i. e. a substituent or moiety with characteristic chemical reactivity. The same functional group will undergo the same or similar chemical reactions regardless of the rest of the modified toxin molecule. This enables systematic prediction of chemical reactions and behaviour of the modified toxin in chemical conjugation to an antibody. Preferably, the organic coupling group is capable of, or configured for, a bioorthogonal chemical coupling reaction such as a click chemistry reaction. Preferably, the organic coupling group is selected from the group consisting of amino group, carboxyl group, hydroxyl group, azido group, alkyne group, alkene group, tetrazine group, thiol group, aldehyde group, keto group, oxime, hydrazine, hydrazone, phosphine, phosphonate, and any combination thereof.

A second aspect of the present invention relates to a modified cyanobacterial or fungal toxin obtainable by the method described above.

The modified cyanobacterial or fungal toxin comprises a base structure having a cytotoxicity for a target cell after uptake by a transporter protein of the target cell, and at least one structural modification of the base structure. Herein, the at least one structural modification comprises an organic inhibitory group or effector molecule reducing the uptake of the modified toxin by the transporter protein into the target cell in comparison to the base structure, and an organic coupling group configured for covalent conjugation of the modified toxin to an antibody.

In preferred embodiments, the toxin or the base structure, respectively, is a microcystin having the general structure
cyclo (-A¹-X²-A³-Z⁴-A⁵-A⁶-A⁷), wherein, independently from each other, A¹ and A³ each represent a D-amino acid, A⁵ is selected from the group consisting of Adda, DM-Adda, dm-Adda, (6Z)Adda, and ADM-Adda, A⁶ is selected from the group consisting of D-Glu and D-Glu(OCH₃), A⁷ is selected from the group consisting of Mdha, MdhB, Dha, L-Ser, L-MeSer, Dhb, (E)-Dhb, (Z)-Dhb, MeLan, Cys and Thr or a modified L-amino acid, and X² and Z⁴ are, independently from each other, an L-amino acid. In some embodiments, A¹ is selected from the group consisting of D-Ala, D-Leu and D-Ser or a modified D-amino acid. In some embodiments, A³ is selected from the group consisting of D-Asp and D-MeAsp or a modified D-amino acid. D-MeAsp is D-erythro-β-methylaspartic acid, Mdha is N-methyldehydroalanine, Mdhb is N-methyldehydrobutyrate. Adda is 3-amino-9-methoxy-2,6,8-trimethyl-10-phenyl-deca-4,6-dienoic acid, ADM-Adda is O-acetyl-9-O-desmethyl-Adda, DM-Adda is 9-O-desmethyl-Adda, dm-Adda is Adda demethylated at C-2, C-6, or C-8, Dha is dehydroalanine, Dhb is dehydrobutyrine, MeLan is N-methyl-lanthionin. As used herein, a "modified amino acid" is an amino acid with a side-chain that is not present in a natural counterpart of the microcystin or the base structure, respectively, which comprises a chemical group covalently conjugated to, or a functional group configured for covalent conjugation to, the at least one structural modification. A modified amino acid may for example be selected from the group consisting of Azidonorvaline, Propargyltyrosine, Azidolysine Azidophenylalanine, Propargylcysteine, Propargylserine, Azidoalanine, or any other amino acid not present in a corresponding natural cyanobacterial or fungal toxin or base structure and with a functional group configured for, or capable of, covalent conjugation.

Microcystins are potent inhibitors of type 1 and type 2A protein phosphatases. Protein phosphatases 1 (PP1) and 2A (PP2A) are two of the major phosphatases in eukaryotic cells that dephosphorylate serine and threonine residues. For example, the IC50 of naturally occurring microcystin-LR is 0.03 nM for type 1 protein phosphatase and 0.04 nM for type 2A protein phosphatases. It is established in the art that positions A⁵ an A⁶ are essential for the inhibitory effect of microcystins against PP1 and PP2A. Therefore, the at least one structural modification of the microcystin is preferably provided in position A¹, X², A³, Z⁴ and/or A⁷. Preferentially, the at least one structural modification is provided in position A¹, X², Z⁴ and/or A⁷, most preferred in position X² or Z⁴. For example, the at least one structural modification is incorporated in, or covalently bound to, the side-chain of the amino acid in said position. Accordingly, the base structure may comprise a modified amino acid in at least one of positions A¹, X², A³, Z⁴ and/or A⁷. Preferentially, the at least one structural modification is provided in position A¹, X², Z⁴ and/or A⁷, most preferred in position X² and/or Z⁴. Preferably, the at least one structural modification is provided in at most two positions, for example in exactly one position.

In other preferred embodiments, the toxin or the base structure, respectively, is a nodularin having the general structure
cyclo [-A¹-*A²-A³-A⁴-*A⁵],
wherein, independently from each other, A¹ is D-Asp or D-MeAsp or a modified D-amino acid, A² is Arg or Har or a modified L-amino acid, A³ is selected from the group consisting of Adda, DM-Adda, (6Z)Adda, and MeAdda, A⁴ is selected from the group consisting of D-Glu and D-Glu(OCH₃), A⁵ is Dhb or Mdhb or a modified L-amino acid, and "_{*}" represents an isopeptide bond.

It is established in the art that positions A³ an A⁴ are essential for the inhibitory effect of nodularins against PP1 and PP2A. Accordingly, the at least one structural modification of the nodularin is preferably provided in position A¹, A² and/or A⁵. For example, the at least one structural modification is incorporated in, or covalently bound to, the side-chain of the amino acid in said position. Accordingly, the base structure may comprise a modified amino acid in at least one of positions A¹, A² and/or A⁵. Preferably, the at least one structural modification is provided in at most two positions, for example in exactly one position. Preferentially, the at least one structural modification is provided in position A².

In yet other preferred embodiments, the toxin or the base structure, respectively, is an amatoxin having the general structure
cyclo {A¹-cyclo [A²-A³-A⁴-A⁵-A⁶] -A⁷-A⁸} (SEQ ID NO:1),
wherein, independently from each other, A¹ is Isoleucin (Ile), Hydroxyisoleucin (Hyile) or Dihydroxyisoleucin (Dihyile), A² is Tryptophan (Trp) or Hydroxytryptophan (Hytrp), A³ is Glycin (Gly), A⁴ is Isoleucin (Ile), A⁵ is Glycin (Gly), A⁶ is Cystein (Cys), A⁷ is Asparagin (Asn) or Aspartate (Asp) and A⁸ is Prolin (Pro) or Hydroxyprolin (Hypro), with cross-linking between A² and A⁶ via a sulfoxide (S=O) bridge.

For example, the amatoxin can be one of the group consisting of α-amanitin, β-amanitin, γ-amanitin, δ-amanitin, ε-amanitin, amanullin, amanullinic acid, amaninamide, amanin and proamanullin. Preferably, the amatoxin is an amanitin.

In preferred embodiments, the at least one structural modification of the amatoxin or amanitin, respectively, is provided in position A² Hydroxytryptophan (Hytrp) or A⁸ Hydroxyprolin (Hypro). For example, the at least one structural modification is incorporated in, or covalently bound to, the side-chain of the amino acid in said position, e. g. via the hydroxy group or amino group in the side-chain. Preferentially, the at least one structural modification is provided in position A² Hydroxytryptophan (Hytrp).

The potency of a toxin generally results from the correlation of cell uptake and the inhibitory effect, which can be, for example, protein phosphatase inhibition. The correlation can be expressed, for example, in the form of an IC50 value, which corresponds to the toxin concentration needed to kill 50% of cells exposed to the toxin, hereinafter termed IC50_cytotox. However, given that the IC50_cytotox value is not only dependent on cell uptake but also on the level of the inhibitory effect, a high IC50_cytotox value can be caused either by a poor cell uptake or by a poor inhibitory effect of the toxin, or both, whereas a low IC50_cytotox value can be caused by high cell uptake or by a strong inhibitory effect or both, respectively. The inventors therefore recognized that a more suitable characteristic of the cytotoxic property of modified toxins is expressed in the relation between the IC50_cytotox value and the inhibitory concentration of the toxin needed to achieve half maximal inhibitory effect, e. g. to inhibit the protein phosphatase by 50%, hereinafter referred to as IC50_PPI. Accordingly, if a ratio of IC50_cytotox/IC50_PPI is greater for a modified toxin than for the base structure, cell uptake is impaired relative to inhibitory function, which is a preferred characteristic of the modified toxins of the present invention. Therefore, preferred embodiments of the methods described herein comprise determining a ratio IC50_cytotox/IC50_PPI of the modified toxin and/or of the base structure and optionally comparing or correlating the IC50_cytotox/IC50_PPI of the modified toxin with the IC50_cytotox/IC50_PPI of the base structure. Preferably, the IC50_cytotox/IC50_PPI ratio of the modified toxin is at least twice as high, more preferably at least three times or at least four times as high, particularly preferred at least five times as high as the IC50_cytotox/IC50_PPI ratio of the base structure. Methods for determining the IC50 value of an inhibitor are well known in the art. In this regard, references also made to the detailed description of embodiments below.

In certain embodiments, the least one structural modification comprises a group B-In, wherein the group In denotes the inhibitory group or the effector molecule reducing the uptake of the toxin by the transporter protein into the target cell and wherein the group B denotes a bridging group covalently bridging the group In to the toxin or base structure, respectively. Preferably, the group B comprises a group selected from a triazole, a peptide bond, a disulfide, a C₁ to C₁₀-alkylgroup, a C₁ to C₁₀-alkylarylgroup, an (oxyethylene)₁₋₁₀ group, or any combination thereof. In preferred embodiments, the group B comprises a triazole.

In some embodiments, the group In is selected from a group consisting of *-C₁-C₁₀alkyl-NH₃⁺, *-C₁-C₁₀alkyl-CO₂⁻, *-C₁-C₁₀alkyl-CH(NH₃⁺)-CO₂⁻, *-C₁-C₁₀alkyl-CH(NH₃⁺)-C₁-C₄alkyl-CO₂⁻, *-C₁-C₁₀alkyl-O-C(O)-NH-C₁-C₆alkyl-CH(NH₃⁺)-C₁-C₄alkyl-CO₂⁻, *-C₁-C₁₀alkyl-NH-CHO, *-C₁-C₁₀alkyl-OH, *-C₁-C₁₀isoalkyl-CH(NH₃⁺)-CO₂⁻, *-C₁-C₄alkyl-NH-Biotin, *-C₁-C₄alkyl-(O-CH₂-CH₂)₁₋₁₀-NH-Biotin, *-C₁-C₄alkyl-(PEG)₂₋₁₀-NH-Biotin, *-C₁-C₄alkyl-NH₂⁺-C₁-C₆alkyl-NH₂⁺-C₁-C₆alkyl-NH₃⁺, *-Phenyl-C₁-C₄alkyl-CH(NH₃⁺)-CO₂⁻, 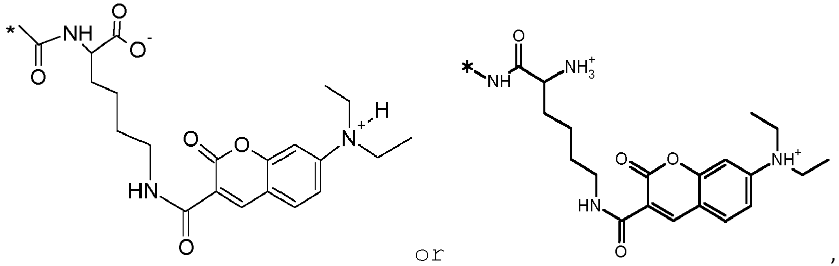 wherein the "*" denotes the point of covalent attachment to the bridging group B and "+" and "-" indicate charges at physiological pH-value.

In certain embodiments, the at least one structural modification can also include a label for in-vivo visualization, such as a fluorescence label or a near infrared dyes, for example, which allow deep-tissue imaging in-vivo by using luminescent phenomena in the near infrared region.

As already described above, the at least one structural modification can comprise a first structural modification comprising the organic inhibitory group or effector molecule reducing the uptake of the toxin by the transporter protein into the target cell in comparison to the base structure, i. e. the toxin lacking the first structural modification, and, separately, a second structural modification comprising the organic coupling group configured for covalent conjugation of the modified toxin to an antibody. In particular, the first and second structural modification can be provided in different positions of the microcystin, nodularin, or amatoxin, respectively. For example, the base structure can be a microcystin, wherein the first structural modification is provided in position X² and the second structural modification is provided in one of positions A¹, A³, Z⁴ or A⁷, or vice versa. Alternatively, the first structural modification can be provided in position Z⁴ and the second structural modification can be provided in one of positions A¹, X² A³ or A⁷, or vice versa. Preferably, one of the first and second structural modification is provided in position X² and the other structural modification is provided in position Z⁴. As noted above, the first and second structural modification can, independently of each other, be attached to, or incorporated in, a side-chain of the amino acid in said position. In some embodiments, the first structural modification is covalently attached to a side-chain of the amino acid, whereas the second structural modification is incorporated in a side-chain of the amino acid, for example by incorporating a modified amino acid having a side-chain that includes the second structural modification, as described in WO 2018/206715 A2. In particular, the second structural modification can comprise an alkyne group or an azido group, or any of the groups configured for covalent coupling of the modified toxin to an antibody described above.

In some embodiments, the second structural modification can comprise a linker which incorporates the organic coupling group configured for covalent conjugation of the modified toxin to an antibody, or is arranged between the side-chain of the amino acid and the organic coupling group.

In accordance with the ordinary understanding in the field of ADCs, a "linker" is a bifunctional or multifunctional moiety which can be used to covalently bridge the modified toxin with an antibody to form an antibody-drug conjugate. There are two genera of linkers, namely so-called "cleavable linkers" and "non-cleavable linkers". A non-cleavable linker is essentially stable outside a cell, i. e. extracellularly. A cleavable linker includes a pre-defined cleavage site which is cleavable by enzymatic activity, hydrolysis, or other metabolic conditions. A linker may also include a spacer structure that spatially separates the modified toxin or base structure, respectively, from the antibody. The use of linkers is well-known in the art and a skilled artisan is readily capable to select a suitable linker based on his knowledge and this disclosure.

For example, the linker may comprise an organic coupling group that is configured for covalent coupling to a cysteine thiol, an amine, e. g. N-terminus or amino acid side chain such as lysine, or any other modification of the antibody, as described below. In some preferred embodiments, the linker is a non-cleavable linker. The linker may be substituted with a sulfonate substituent or other substituents which may increase water solubility of the reagent and facilitate the coupling reaction of the linker reagent with the antibody. The organic coupling group may be configured for covalent coupling to a nucleophilic group on an antibody, including, but are not limited to, N-terminal amino groups, side-chain amino groups, e. g. of lysine, side-chain thiol groups, e. g. of cysteine, and hydroxyl groups or amino groups of a carbohydrate in case of a glycosylated antibody. Amino groups, thiol groups and hydroxyl groups are nucleophilic and capable of reacting to form covalent bonds with e. g. an electrophilic group of the linker, including, but not limited to, active esters such as NHS esters, HOBt esters, haloformates, and acid halides, alkyl and benzyl halides such as haloacetamides, aldehydes, ketones, carboxyl and maleimide groups. Certain antibodies have reducible interchain disulfides, i. e. cysteine bridges. Antibodies may be made reactive for conjugation with the linker by treatment with a reducing agent such as DTT (dithiothreitol). Additional nucleophilic groups can be introduced into antibodies through the reaction of lysines with 2-iminothiolane (Traut's reagent) resulting in conversion of an amine into a thiol. Reactive thiol groups may be introduced into the antibody by introducing one, two, three, four, or more cysteine residues e. g. by preparing mutant antibodies comprising one or more non-native cysteine residues or through commercially available chemical kits.

In some embodiments, the second structural modification, in particular the organic coupling group, is configured for being connected to an antibody via a click chemistry reaction. A click chemistry reaction in the sense of the present invention is for example Copper(I)-catalyzed azide-alkyne cycloaddition, strain-promoted azide-alkyne cycloaddition, strain-promoted alkyne-nitrone cycloaddition, alkene and azide [3+2] cycloaddition, alkene and tetrazine inverse-demand Diels-Alder reaction, alkene and tetrazole photoclick reaction. Accordingly, the organic coupling group is preferentially an azide, an alkyne, an alkene, or a tetrazine.

In some specific embodiments, the at least one structural modification is one single structural modification, i. e. the organic inhibitory group comprises, or consists of, the organic coupling group or a part thereof, or vice versa. In some preferred embodiments, the organic coupling group forms at least part of the organic inhibitor group, so that at least a part of the organic inhibitor group is also configured for covalent conjugation of the modified toxin to an antibody. In this way, the function of organic inhibitory group can be intentionally impaired, at least partially, when the modified toxin is covalently conjugated through the organic coupling group to an antibody in an ADC. However, upon cleavage of the conjugation bond, either intracellularly or extracellularly, the organic inhibitor group is restored and prevents subsequent internalization of the released modified toxin by healthy cells. In this way, enhancement of both the internalization of the modified toxin when coupled to an antibody and inhibition of internalization of the modified toxin after release from the antibody is achieved. In some embodiments hereunder, the single structural modification comprises a functional group as the organic coupling group configured for being covalently connected to the antibody, wherein the functional group is selected from a group consisting of **-NH₃⁺, **-COO₂⁻, or **-OH, wherein the "**" denotes the point of attachment to the remainder of the single structural modification and "+" and "-" indicate charges at physiological pH-value.

As noted above, the base structure can be a naturally occurring toxin or non-naturally occurring. The latter can for example be a base structure of a naturally occurring toxin that has been modified with at least one functional group configured for being connectable by chemical conjugation to a modifying molecule, wherein a chemical conjugation reaction between the functional group and the modifying molecule creates the at least one structural modification. In preferred embodiments, the at least one functional group is an azido group or an alkyne group. However, the functional group can also be any of the functional groups disclosed above.

The modified toxin described above can be advantageously used as a cytotoxic drug in an antibody-drug conjugate for reducing the uptake of the toxin by the transporter protein into the cell in comparison to a toxin having the base structure without the at least one structural modification.

Accordingly, a third aspect of the present invention relates to a use of a modified toxin as described above in manufacturing an antibody-drug conjugate, wherein the use comprises covalently conjugating the modified toxin to an antibody or a fragment thereof comprising an antigen-specific binding site, thereby forming the antibody-drug conjugate.

Accordingly, a fourth aspect of the present invention provides an antibody-drug conjugate comprising a modified toxin as described above as the drug and an antibody or a fragment thereof comprising an antigen-specific binding site, wherein the antibody or the fragment thereof is attached, i. e. covalently conjugated, to the modified toxin via the organic coupling group.

In some embodiments hereunder, the at least one structural modification is one single structural modification and the antibody is attached to the single structural modification via one of an amide, ester, ether, triazole or carbamate linkage, for example.

For the purpose of the present invention, the term "antibody" is used in its broadest sense and encompasses monoclonal antibodies, polyclonal antibodies, dimers, multimers, bispecific antibodies or multispecific antibodies, as well as antibody fragments having an antigen-specific binding site, i. e. an antigen binding site that immuno-specifically binds an antigen of a target cell or a part thereof. Examples of antibody fragments include Fab, Fab', F(ab')2, and scFv fragments, single-domain antibodies, also known as a nanobodies, diabodies, linear antibodies, fragments produced by a Fab expression library, anti-idiotypic (anti-Id) antibodies, CDR (complementary determining region), and epitope-binding fragments of any of the above which immuno-specifically bind to e. g. cancer cell antigens, viral antigens or microbial antigens, single-chain antibody molecules, multispecific antibodies formed from antibody fragments. Antibodies may be murine, human, humanized, chimeric, or derived from other species. The antibody may be any type of immunoglobulin, e. g. IgG, IgE, IgM, IgD, or IgA, any class, e. g. IgGl, IgG2, IgG3, IgG4, IgA1 or IgA2, or subclass of immunoglobulin.

In preferred embodiments, the antibody or fragment thereof is specific for a tumor-associated antigen (TAA), i. e. an anti-TAA antibody, or a tumor-specific antigen (TSA), i. e. an anti-TSA antibody. Tumor-specific antigens are found on cancer cells only, not on healthy cells. Tumor-associated antigens have elevated levels on tumor cells, but are also expressed at lower levels on healthy cells. Such tumor-associated antigens and tumor-specific antigens are known in the art. Examples of TAAs and TSAs include, but are not limited to, BMPR1B (bone morphogenetic protein receptor-type IB, Genbank accession no. NM-001203), E16 (LAT1, SLC7A5, Genbank accession no. NM-003486), STEAP1 (six transmembrane epithelial antigen of prostate, Genbank accession no. NM-012449), 0772P (CA125, MUC16, Genbank accession no. AF361486), MPF (MPF, MSLN, SMR, megakaryocyte potentiating factor, mesothelin, Genbank accession no. NM-005823), Napi3b (NAPI-3B, NPTIIb, SLC34A2, solute carrier family 34 (sodium phosphate), member 2, type II sodium-dependent phosphate transporter 3b, Genbank accession no. NM-006424), Sema 5b (F1110372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878), PSCA hlg (2700050C12Rik, C530008O16Rik, RIKEN cDNA 2700050C12, RIKEN cDNA 2700050C12 gene, Genbank accession no. AY358628), ETBR (Endothelin type B receptor, Genbank accession no. AY275463), MSG783 (RNF124, hypothetical protein F1120315, Genbank accession no. NM-017763), STEAP2 (HGNC-8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138), TrpM4 (BR22450, F1120041, TRPM4, TRPM4B, transient receptor potential cation channel, subfamily M, member 4, Genbank accession no. NM-017636), CRIPTO (CR, CR1, CRGF, CRIPTO, TDGF1, teratocarcinoma-derived growth factor, Genbank accession no. NP-003203 or NM-003212), CD21 (CR2 (Complement receptor 2) or C3DR(C3d/Epstein Barr virus receptor) or Hs.73792 Genbank accession no. M26004), CD79b (CD79B, CD79β, IGb (immunoglobulin-associated beta), B29, Genbank accession no. NM-000626 or 11038674), FcRH2 (IFGP4, IRTA4, SPAP1A (SH2 domain containing phosphatase anchor protein 1a), SPAP1B, SPAP1C, Genbank accession no. NM-030764, AY358130), HER2 (ErbB2, Genbank accession no. M11730), NCA (CEACAM6, Genbank accession no. M18728), MDP (DPEP1, Genbank accession no. BC017023), IL20Ra (IL20Ra, ZCYTOR7, Genbank accession no. AF184971), Brevican (BCAN, BEHAB, Genbank accession no. AF229053), EphB2R (DRT, ERK, HekS, EPHT3, Tyro5, Genbank accession no. NM-004442), ASLG659 (B7h, Genbank accession no. AX092328), PSCA (Prostate stem cell antigen precursor, Genbank accession no. AJ297436), GEDA (Genbank accession No. AY260763), AAP14954 lipoma HMGIC fusion-partner-like protein/pid=AAP14954.1 Homo sapiens (human)), BAFF-R (B cell-activating factor receptor, BLyS receptor 3, BR3, Genbank accession No. AF116456), BAFF receptor/pid=NP-443177.1-Homo sapiens, CD22 (B-cell receptor CD22-B isoform, BL-CAM, Lyb-8, Lyb8, SIGLEC-2, FLJ22814, Genbank accession No. AK026467), CD79a (CD79A, CD79α, immunoglobulin-associated alpha), CXCR5 (Burkitt's lymphoma receptor 1), HLA-DOB (Beta subunit of MHC class II molecule), P2X5 (Purinergic receptor P2X ligand-gated ion channel 5), CD72 (B-cell differentiation antigen CD72, Lyb-2, Genbank accession No. NP-001773.1), LY64 (Lymphocyte antigen 64 (RP105)), FcRH1 (Fc receptor-like protein 1), IRTA2 (FcRH5, Immunoglobulin superfamily receptor translocation associated 2), TENB2 (TMEFF2, tomoregulin, TPEF, HPP1, TR), MUC1 (Tumor-associated MUC1 glycopeptide epitopes).

In preferred embodiments, the antibody-drug conjugate has a drug-to-antibody-ratio (DAR) of 2-12, i. e. at least two and at most twelve modified toxins bound per antibody. Preferably, the DAR is 2-10, more preferably 2-8, most preferred 4-8.

A fifth aspect of the present invention provides a modified cyanobacterial or fungal toxin or an antibody-drug conjugate as described above for use in the treatment of a malignant disease.

A sixth aspect of the present invention provides a method for treating a human subject afflicted with a malignant disease, comprising administering to the subject a therapeutically effective dosing regimen of the antibody-drug conjugate defined above.

In certain embodiments, the malignant disease is cancer. Examples of cancer to be treated herein include, but are not limited to, carcinoma, lymphoma, blastoma, sarcoma, and leukemia or lymphoid malignancies. More particular examples of such cancers include squamous cell cancer, e. g. epithelial squamous cell cancer, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, gastrointestinal stromal tumor (GIST), pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. The cancer can be characterized by overexpression of HER2 or ErbB receptor, for example.

In a seventh aspect, the present invention provides a method for manufacturing a modified cyanobacterial or fungal toxin as described above.

The method comprises the following steps: A) providing a toxin having a base structure known to be cytotoxic for a target cell after uptake by a transporter protein of the target cell, B) introducing the at least one structural modification into the toxin or the base structure, respectively, thereby forming the modified toxin.

In some embodiments, the toxin or the base structure, respectively provided in step A) comprises at least one first functional group configured for and/or capable of covalent conjugation, wherein in method step B) the at least one structural modification is introduced into the toxin or the base structure, respectively, via covalent conjugation to the at least one first functional group.

In some embodiments, method step B) comprises introducing at least a first and a second structural modification into the toxin or the base structure, respectively, wherein the first structural modification comprises the organic inhibitory group and the second structural modification comprises the organic coupling group configured for covalent conjugation of the modified toxin to an antibody.

Furthermore, method step B) can comprise providing a modifying molecule, wherein the modifying molecule comprises the organic inhibitory group or effector molecule, respectively, and/or the organic coupling group, and covalently conjugating the modifying molecule to the at least one first functional group, thereby creating the at least one structural modification.

The modifying molecule can comprise a second functional group which is mutually reactive with the first functional group of the toxin or base structure provided in step A). In particular, the first and second functional group can be mutual reaction partners in any one of the click chemistry reactions described above, such as an azide or tetrazine and an alkyne or alkene, for example. The first and second functional group can however also be selected from any of the other mutually reactive functional groups described above, such as amino group and carboxylic group, aldehyde group or keto group, for example.

Moreover, it is understood that the various embodiments described for one aspect of the invention are also applicable to the other aspects of the invention. Accordingly, features and functions disclosed above and below in connection with the method of modifying a cyanobacterial or fungal toxin may therefore also relate to the modified cyanobacterial or fungal toxin, the use of a modified toxin in manufacturing an antibody-drug conjugate, the method for manufacturing a modified cyanobacterial or fungal toxin, the antibody-drug conjugate as well as its medical use, and vice versa.

### Brief description of figures

- Fig. 1: Structural formula of MC-(Azidonorvaline)R (A) and MC-Propargyltyrosine)R (B) used as reference toxins with cytotoxic base structure;
- Fig. 2: Structural formula of modified toxin MC-(AznvaPrgam)R produced by modifying MC-(Azidonorvaline)R with propargylamine in accordance with the invention;
- Fig. 3: Scheme of structural modification of MC-Propargyltyrosine)R in accordance with the present invention;
- Fig. 4: Scheme of another structural modification of MC-Propargyltyrosine)R in accordance with the present invention.

Detailed description of embodiments

The present invention is explained in more detail below on the basis of exemplary embodiments with reference to the attached figures. Neither the examples nor the figures shall be considered limiting.

### Example 1: Production of MC-(Azidonorvaline)R and MC-Propargyltyrosine)R as reference toxins with cytotoxic base structures

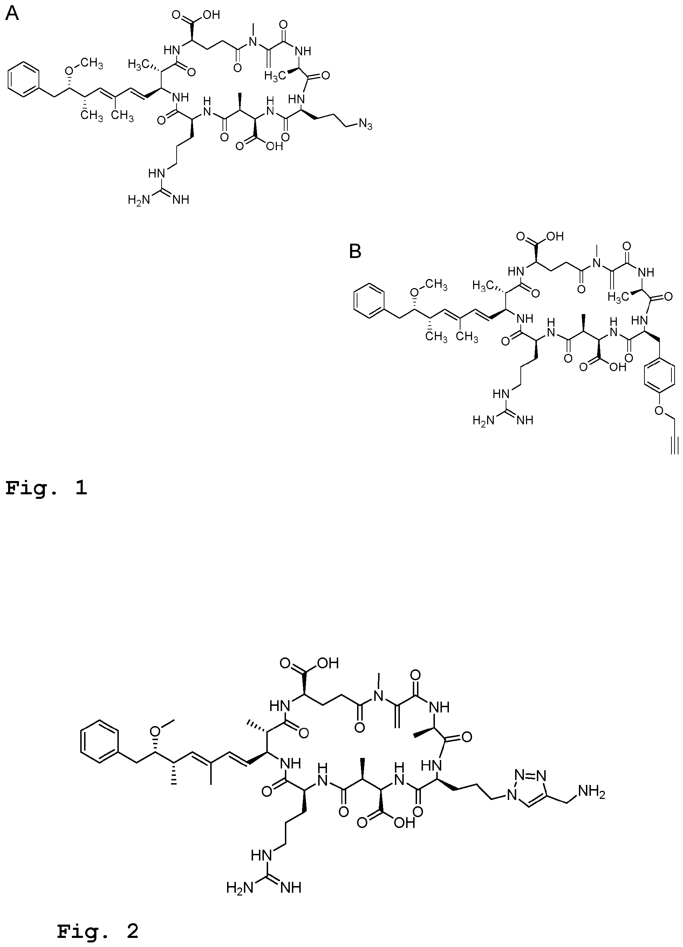

Two reference toxins were produced which have a base structure known to be cytotoxic against OATP-expressing cancer cell lines, namely the microcystins MC-(Azidonorvaline)R or "MC-AznvaR" shown in Fig. 1A and MC-(Propargyltyrosine)R or "MC-PrtyrR" shown in Fig. 1B. Each base structure contains a functional group configured for covalent coupling of a modifying moiety via click chemistry in position X², namely an azide in MC-AznvaR and an alkyne in MC-PrtyrR. Accordingly, the base structures can be written as cyclo(D-Ala-Aznva-D-Asp-R-Adda-D-Glu-Mdha) for MC-AznvaR (SEQ ID NO:2) and cyclo(D-Ala-Prtyr-D-Asp-R-Adda-D-Glu-Mdha) for MC-PrtyrR (SEQ ID NO:3).

MC-(Aznva)R and MC-(Prtyr)R were produced essentially as described in WO 2018/219619 A1. Briefly, a cyanobacterial strain producing natural microcystins was cultivated under conditions allowing for growth of the strain. During the cultivation, the inorganic cultivation medium was supplemented with either the modified amino acid Azidonorvaline (Aznva) or Propargyltyrosine (Prtyr). Those modified amino acids represent modified building blocks of the position X² of the microcystins produced by the strain and have been incorporated instead of the natural amino acid substrate into the microcystins during the cultivation of the strain, thereby resulting in the biosynthesis of a non-naturally occurring microcystin with the respective modified amino acid in position X². After the cultivation, the biomass was harvested and extracted and the non-natural microcystins serving as base structures were purified by means of HPLC.

In the following, both base structures were modified with structural modifications comprising an organic inhibitory group and an organic coupling group in accordance with the present invention as described below.

### Example 2: Structural modification of MC-AznvaR and MC-PrtyrR using copper-catalyzed azide-alkyne-cycloaddition (CuAAC)

Several different structural modifications were introduced into MC-AznvaR and MC-PrtyrR in position X² via copper-catalyzed azide-alkyne-cycloaddition (CuAAC) to provide various modified toxins in accordance with the present invention.

The following modifying molecules were used for the structural modification of MC-AznvaR:
Propargyllysine (Prglys) as one example of a structural modification with an amphoteric organic inhibitory group and an amino group and a carboxyl group as organic coupling group;
Biotinalkyne (Albio) as one example of a structural modification with a heterocyclic inhibitory group and biotin as organic coupling group;
Propargylamine (Prgam) as one example of a structural modification with an inhibitory group carrying a positive charge at physiological pH and an amino group as organic coupling group.

The following modifying molecules were used for the structural modification of MC-PrtyrR:
Azidoethanol (Azeth) as one example of a structural modification with an alcohol as inhibitory group and a hydroxy group as organic coupling group;
Biotin-PEG-3-Azide as one example of a structural modification with a heterocyclic inhibitory group and biotin as organic coupling group;
Azidohomoalanine (AzHala) as one example of a structural modification with an amphoteric organic inhibitory group and an amino group and a carboxyl group as organic coupling group;
Azidophenylalanine (AzPhe) as another example of a structural modification with an amphoteric organic inhibitory group and an amino group and a carboxyl group as organic coupling group;
N1-Azidospermin (AzSpe) as another example of a structural modification with an inhibitory group carrying a positive charge at physiological pH and an amino group as organic coupling group;
Azidopropylformamid (AzProam) as one example of a structural modification with an organic formamide as inhibitory group and organic coupling group;
Azidoacetic acid (Azacac) as another example of a structural modification with an inhibitory group carrying a negative charge at physiological pH and a carboxyl group as organic coupling group;
2-Amino-3-Azidobutanoic acid (AzAbu) as another example of a structural modification with an amphoteric organic inhibitory group and an amino group and a carboxyl group as organic coupling group;
3-Amino-4-Azidobutyric acid (AzDbu) as another example of a structural modification with an amphoteric organic inhibitory group and an amino group and a carboxyl group as organic coupling group.

In each case, CuAAC was performed with either MC-PrtyrR (0.32 mg, 0.3 µmol, 1 eq) or MC-AznvalR (0.31 mg, 0.3 µmol, 1 eq) in 300 µL DMSO and 150 mL of the "clickable" modifying molecules described above (10 mM, 1.5 µmol, 5 eq.) using 150 µL DMSO, 930 µl H₂O, 1.5 µL 100 mM CuSO₄ solution (0.5 eq), 3.75 µL 200 mM tris(3-hydroxypropyltriazolylmethyl)amine (THPTA) solution (2.5 eq.), 75 µL 100 mM aminoguanidine (25 eq.).

The reaction was started by adding 37.5 µL 100 mM sodium ascorbate solution (12.5 eq.). The modified toxins were isolated either by C-18 solid phase extraction (SPE) or semi-preparative C-18 reverse phase (RP)-HPLC and lyophilized.

Fig. 2 exemplarily shows the structural formula of the modified toxin MC-(AznvaPrgam)R which was obtained by modifying MC-(Azidonorvaline)R with propargylamine in accordance with the invention.

### Example 3: Synthesis of modified MC-PrtyrR compound 1

In accordance with the present invention, MC-PrtyrR was modified with azidoacetyl-L-Lys(7-DCCA)-OH as one example of a heterocyclic, at physiological pH negatively charged structural modification, wherein the organic coupling group is a carboxyl group. The structural modification also contains a fluorescence structure. The synthesis route is shown in Fig. 2 and is described in more detail below.

Synthesis of Boc-L-Lys(7-DCCA)-OH (1a): 7-(Diethylamino)coumarin-3-carboxylic acid *N*-succinimidyl ester (7-DCCA-NHS; 10 mg, 27.9 µmol, 1 eq.) and Boc-L-lysine (10.3, 41.9 µmol, 1.5 eq) were dissolved in 300 µL *N,N-*dimethylformamide (DMF) each and combined followed by addition of 400 µL 0.1 M sodium phosphate buffer pH 8. The reaction mix was stirred for 6 hours at RT. Reaction product 1a was isolated by C-18 solid phase extraction (SPE) and lyophilized. The reaction product has a molecular structure of C₂₅H₃₅N₃O₇ and a monoisotopic mass of 489,2475 g/mol.

Synthesis of H-L-Lys(7-DCCA)-OH (1b): Compound 1a (3.5 mg, 7.15 µmol, 1 eq.) was dissolved 700 µL dichlormethane (DCM) followed by addition of 300 µL trifluoroacetic acid (TFA). The reaction was stirred for 2 hours at RT. The solvent was evaporated and the remaining solid was triturated with diethyl ether. The precipitate was isolated by centrifugation affording compound 1b with a molecular structure of C₂₀H₂₇N₃O₅ and a monoisotopic mass of 389,1951 g/mol.

Synthesis of azidoacetic acid *N*-succinimidyl ester (1c): *N-*Hydroxysuccinimide (NHS; 62.6 mg, 0.54 mmol, 1.1 eq.) was dissolved in 750 µL tetrahydrofuran (THF) and azidoacetic acid (37 µL, 0.49 mmol, 1 eq.) was added. N,N'-Dicyclohexylcarbodiimide (DCC; 112.3 mg, 0.54 mmol, 1.1 eq.) was dissolved in 750 µL THF and dropwise added to the reaction mixture. After 17 hours, the reaction mixture was separated by suction filtration and the filtrate was evaporated. To remove further impurities, the product was resolved in 20 mL DCM and extracted twice with 10 mL saturated NaHCO₃ and 50 mL brine. The organic layer was dried with Na₂SO₄. The filtrate was evaporated affording 1c with a molecular structure of C₂₂H₂₈N₆O₆ and a monoisotopic mass of 472,2070 g/mol.

Synthesis of azidoacetyl-L-Lys(7-DCCA)-OH (1d): Compound 1b (2.8 mg, 7.2 µmol, 1 eq.) was dissolved together with 1c (4.28 mg, 21.6 µmol, 3 eq.) in 500 µL DMF. Then, 500 µL 0.1 M sodium phosphate buffer pH 8 was added and the reaction was stirred for 16 hours at RT. Compound 1d with a molecular structure of C₂₂H₂₈N₆O₆ and a monoisotopic mass of 472,2070 g/mol was isolated by semi-preparative C-18 RP-HPLC and lyophilized.

Synthesis of Compound 1 by CuAAC: The copper-catalyzed azide-alkyne-cycloaddition (CuAAC) was performed with MC-PrtyrR (0.25 mg, 0.23 µmol, 1 eq.) and 1d (0.16 mg. 0.35 µmol, 1.5 eq.) using 350 µL DMSO, 75 µl H₂O, 25 µL 100 mM CuSO₄ solution (5 eq), 25 µL 200 mM THPTA solution (10 eq.). The reaction was started by adding 25 µL 100 mM sodium ascorbate solution (5 eq.). The reaction product compound 1 with the chemical structure C₇₇H₁₀₂N₁₆O₁₉ and a monoisotopic mass of 1554, 7507 g/mol was isolated by semi-preparative C-18 RP-HPLC and lyophilized.

### Example 4: Synthesis of modified MC-PrtyrR compound 2

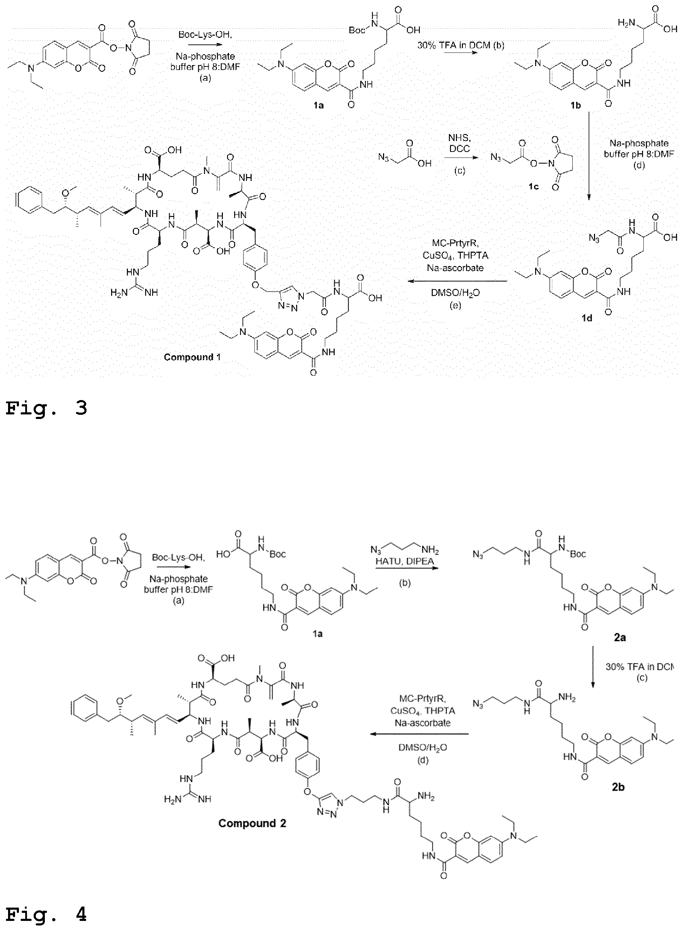

In accordance with the present invention, MC-PrtyrR was modified with H-L-Lys(7-DCCA)-azidopropyl amid as one example of a heterocyclic, at physiological pH positively charged structural modification, wherein the organic coupling group is an amino group. The structural modification also contains a fluorescence structure. The synthesis route is shown in Fig. 3 and is described in more detail below.

Synthesis of Boc-L-Lys(7-DCCA)-azidopropyl amid (2a): Compound 1a (3 mg, 6.13 µmol, 1 eq.), O-(7-azabenzotriazol-1-yl)-N,N,N',N'-tetramethyluronium-hexafluorphosphat (HATU; 4.66 mg, 12.26 µmol, 2 eq.) and N,N-Diisopropylethylamin (DIPEA; 2.1 µL, 12.26 µmol, 2 eq.) were dissolved in 1 mL DMF and stirred for 15 min in the dark. Then, 3-azidopropanamine (0.7 µL, 6.75 µmol, 1.1 eq.) was added and the solution was stirred for 16 h in the dark. Compound 2a with a molecular structure of C₂₈H₄₁N₇O₆ and a monoisotopic mass of 571,3118 g/mol was isolated by C-18 SPE.

Synthesis of H-L-Lys(7-DCCA)-azidopropyl amid (2b): Compound 2a (2.9 mg, 5.1 µmol, 1 eq.) was dissolved 700 µL DCM followed by addition of 300 µL TFA. The reaction was stirred for 2 hours at RT. The solvent was evaporated and the remaining solid was triturated with diethyl ether. The precipitate was isolated by centrifugation affording compound 2b with a molecular structure C₂₃H₃₃N₇O₄ and a monoisotopic mass of 471,2594 g/mol.

Synthesis of Compound 2 by CuAAC: The copper-catalyzed azide-alkyne-cycloaddition (CuAAC) was performed with MC-PrtyrR (0.25 mg, 0.23 µmol, 1 eq.) and 2b (0.16 mg. 0.35 µmol, 1.5 eq.) using 350 µL DMSO, 75 µl H₂O, 25 µL 100 mM CuSO4 solution (5 eq), 25 µL 200 mM THPTA solution (10 eq.). The reaction was started by adding 25 µL 100 mM sodium ascorbate solution (5 eq.). The reaction product compound 2 with a molecular structure C₇₈H₁₀₇N₁₇O₁₇ and a monoisotopic mass of 1553,8031 g/mol was isolated by semi-preparative C-18 RP-HPLC and lyophilized.

### Example 5: Protocol for cell culture of OATP-expressing cancer cell lines and subsequent cytotoxicity assay

HEK293 cells, stably transfected with expression vectors pcDNA3.1(+)-OATPB1 and pcDNA3.1/Hygro(-)-OATP1B3, and the respective empty vectors pcDNA3.1(+) and pcDNA3.1/Hygro(-) as controls were kindly provided by Prof. Dr. Jörg König (Friedrich-Alexander-University Erlangen-Nürnberg, Germany).

All cell lines were maintained in minimal essential medium, supplemented with 10% heat-inactivated fetal bovine serum, non-essential amino acid mix and 2 mM glutamine at 37 °C and 5% CO₂.

HEK293 OATP1B1+ and the corresponding control cell line were constantly selected with 800 µg/mL geneticin (G418), while 250 µg/mL of hygromycin B were used for the selection of HEK293 OATP1B3+ and its empty vector control.

To determine the cytotoxicity of the modified MC-AznvaR and MC-PrtyrR toxin described in examples 2-4, 5 × 10⁴ cells per well of each cell line were seeded in 96-well plates in triplicates without selection marker. After 24 hours, transporter expression was induced by addition of 10 mM sodium butyrate. On the next day, the medium was removed, and the cells were incubated with eight different concentrations of each of the modified toxins (0.01 nM to 3 µM) and unmodified MC-AznvaR and MC-PrtyrR as controls in medium without selection markers for 48 hours.

Then, the cells were fixed, washed and stained with sulforhodamine B (SRB) as previously described (Vichai et al., Nature protocols 2006, 1, 1112-1116). In brief, 10 µL trichloroacetic acid (10%) were directly added to each well and incubated for 1 h at 4 °C. Then, the solution was removed and each well washed thrice with 200 µL H₂O. Afterwards, the wells were dried and 100 µL 0.057% SRB-solution (in 1% acetic acid) was added per well and incubated for 30 minutes at 4 °C. The solution was removed and each well was washed thrice with 200 µL acetic acid (1%). After drying of the wells, 200 µL Tris buffer (10 mM, pH 10.5) was added and the plates were shaked for five minutes. Then, the absorbance of SRB was measured at 510 nm with a TECAN Infinity TECAN Infinity M PLEX plate reader (Tecan Deutschland GmbH, Crailsheim, Germany). Experiments were performed at least in duplicate.

The cell viability and the IC₅₀ values (IC50_cytotox) were calculated with GraphPad PRISM 6 using a nonlinear regression (sigmoidal dose-response).

### Example 6: Protocol for protein phosphatase inhibition (PPI) assay

The PPI assay was carried out as recently described (Heresztyn et al. Water Res 2001, 35, 3049-3056).

Prior to the measurement, different working solutions have been prepared. The reaction buffer was prepared freshly by combining Tris buffer (250 mM, pH 8,1), magnesium chloride solution (200 mM), manganese chloride solution (10 mM) and bovine serum albumin (BSA) solution (5 mg/ml) (50:26:4:20 (v/v)) and stored on ice. For the substrate solution, the reaction buffer was mixed with para-nitrophenyl phosphate (pNPP) stock solution (60 mM) and DTT solution (20 mM) (5:4:1, (v/v)), whereby the pNPP was prepared freshly. The enzyme dilution buffer was obtained by combining solutions of ethylene glycol-bis-(2-aminoethyl)-N,N,N',N'-tetraacetic acid (EGTA; 0.19 mM, in Tris buffer (80 mM, pH 7.0)), manganese chloride (10 mM), dithiothreitol (DTT; 20 mM) and BSA (5 mg/ml) (65:20:5:10 (v/v)). For the enzyme solution, the protein phosphatase-1 catalytic subunit (a-Isoform from rabbit, Sigma-Aldrich) was diluted with enzyme dilution buffer to 10 units/mL and stored on ice.

The assay was performed in 384-well plates. The diluted modified toxins as well as unmodified MC-AznvaR and MC-PrtyrR (4 µL in dimethyl sulfoxide (DMSO)) were preincubated with enzyme solution (4 µL) at 37 °C for 5 minutes. Then, 40 µL substrate solution were added to each well and incubated at 37 °C for 2 hours. The seven toxin concentrations range from 0.001 nM to 1 µM, while a constant substrate concentration of 20 mM was used per well. As positive control, 4 µL DMSO were used instead of MC solution to follow substrate conversion without PPI. The negative control (blank) contained 4 µL enzyme dilution buffer instead of the diluted enzyme preventing any hydrolysis of the substrate. To determine the PPI, the absorbance was measured at 405 nm with a TECAN Infinity M PLEX plate reader. Experiments were performed at least in duplicate and the IC₅₀ values (IC50_PPI) were calculated with GraphPad PRISM 6 using a nonlinear regression (sigmoidal dose-response).

### Example 7: Bioassay results

The following Tables 1-3 summarize separately for the base structures MC-AznvaR (Table 1) and MC-PrtyrR (Tables 2-3) and the corresponding modified toxins derived therefrom in accordance with the present invention the results from the assays described in Examples 5 and 6 above in terms of the general cytotoxicity (IC50_Cytotox) on the OATP1B1 and OATP1B3 expressing cell lines and the protein phosphatase inhibition assays (IC50_PPI). The tables also show ratios of the IC50_Cytotox and IC50_PPI values as well as ratios of these IC50 values for the modified toxins and their corresponding base structure that were used to evaluate whether the structural modification shows the desired effect. In this context, if the ratio IC50_Cyototox/IC50_PPI of the modified toxin is greater than the ratio IC50_Cyototox/IC50_PPI of the base structure, it means that the therapeutic window of the modified toxin is enhanced with respect to the cell line. Such enhancement of the therapeutic window occurs, as the cell uptake of the modified toxin was reduced relative to the protein phosphatase inhibition and/or the protein phosphatase inhibition of the modified toxin was increased relative to cell uptake. Furthermore, if the ratio of IC50_PPI of the modified toxin and the IC50_PPI of the base structure is <1, it shows that the potency of the cytotoxic mode of action of the modified toxin based on the inhibition of the corresponding protein phosphatases is enhanced compared to the base structure.

The results for the structural modifications of the base structure microcystin MC-AznvaR (Table 1) show that for all modified microcystins according to the present invention the therapeutic windows for both OATP-expressing cell lines could be enhanced compared to the base structure. Furthermore, the PPI of two of the three modified microcystins was improved, as indicated by their lower IC50_PPI compared to the IC50_PPI of the base structure, whereas the IC50_PPI of the third modified microcystin remained unaffected by the structural modification.

The results for the structural modifications of the base structure MC-PrtyrR (Tables 2-3) show that for all modified microcystins according to the present invention the therapeutic windows for at least one OATP-expressing cell line could be enhanced compared to the base structure. Four structural modifications increased the therapeutic window for one of the two OATP expressing cell lines whereas eight of the structural modifications increased the therapeutic window for both OATP expressing cell lines.

Furthermore, the results show that the PPI of three of the modified microcystins according to the present invention could be improved, as indicated by their lower IC50_PPI compared to the IC50_PPI of the base structure.

Accordingly, with the modified toxins according to the invention, an up to 140-fold increase, i. e. more than two orders of magnitude, of the therapeutic window was achieved. These improvements and the advantages associated therewith could not have been expected by a person skilled in the art.

The invention is not limited by the description based on the embodiments. Rather, the invention encompasses every new feature as well as every new combination of features, which in particular includes every combination of features in the patent claims and the description, even if this feature or this combination of features is not explicitly defined in the patent claims, the description or the embodiments.

**Tab. 1: Results from cytotoxicity assays (IC50_Cytotox) and protein phosphatase inhibition assays (IC50_PPI) for the base structure MC-AznvaR (reference) and modified variants thereof. "SE" denotes standard error of at least duplicate experiments.**

| Microcystin (MC) | IC_{50_}cytotox on OATP1B1 expressing cell line + SE [nM] | IC_{50_}cytotox on OATP1B3 expressing cell line + SE [nM] | IC_{50_}PPI + SE [nM] | Ratio of IC_{50_}Cytotox OATP1B1/ IC_{50_}PPI | Ratio of IC_{50_}cytotox OATP1B3/ IC_{50_}PPI | Ratio of IC_{50_}Cytotox OATP1B1 (AznvamodR/ AznvaR) | Ratio of IC_{50_}Cytotox OATP1B3 (AznvamodR/ AznvaR) | Ratio of IC_{50_}PPI (AznvamodR/ AznvaR) |
|---|---|---|---|---|---|---|---|---|
| MC-(Aznva)R (base structure) | 24.1 ± 11.5 | 27.0 ± 1.5 | 46.7 ± 9.2 | 0.52 | 0.58 | | | |
| MC-(AznvaPrglys) R | 546.5 ± 68.4 | 421.6 ± 22.6 | 24.0 ± 16.4 | 22.77 | 17.57 | 22.68 | 15.61 | 0.51 |
| MC-(AznvaAlbio)R | 134.8 ± 6.4 | 293.5 ± 146.4 | 51.3 ± 24.8 | 2.63 | 5.72 | 5.59 | 10.87 | 1.10 |
| MC-(AznvaPrgam)R | 521.3 ± 181.2 | 113.3 ± 20.8 | 7.4 ± 0.3 | 70.45 | 15.31 | 21.63 | 4.20 | 0.16 |

**Tab. 2: Results from cytotoxicity assays (IC50_Cytotox) and protein phosphatase inhibition assays (IC50_PPI) for the base structure MC-(Prtyr)R (reference) and modified variants thereof. "SE" denotes standard error of at least duplicate experiments.**

| Microcystin (MC) | IC_{50_}cytotox on OATP1B1 expressing cell line + SE [nM] | IC_{50_}cytotox on OATP1B3 expressing cell line + SE [nM] | IC_{50_}PPI + SE [nM] | Ratio of IC_{50_}cytotox OATP1B1/ IC_{50_}PPI | Ratio of IC_{50_}cytotox OATP1B3/ IC_{50_}PPI | Ratio of IC_{50_}cytotox OATP1B1 (PrtyrmodR/ PrtyrR) | Ratio of IC_{50_}cytotox OATP1B3 (PrtyrmodR/ PrtyrR) | Ratio of IC_{50_}PPI (PrtyrmodR/ PrtyrR) |
|---|---|---|---|---|---|---|---|---|
| MC-(Prtyr)R (base structure) | 82.2 ± 4.5 | 40.4 ± 36.6 | 8.4 ± 5.4 | 9.79 | 4.81 | | | |
| MC-(PrtyrAzeth)R | 442.1 ±17.7 | 307.6 ± 102.8 | 29.7 ±11.6 | 14.89 | 10.36 | 5.38 | 7.61 | 3.54 |
| MC-(PrtyrAzbio)R | 129.1 ± 6.4 | 558.4 ± 300.8 | 17.7 ± 7.8 | 7.29 | 31.55 | 1.57 | 13.82 | 2.11 |
| MC-(PrtyrAzHala)R | 358.8 ± 223.0 | 248.3 ± 288.1 | 5.3 ± 5.4 | 67.70 | 46.85 | 4.36 | 6.15 | 0.63 |
| MC-(PrtyrAzPhe)R | 259.2 ± 2.2 | 133.5 ± 10.5 | 19.6 ± 17.3 | 13.22 | 6.81 | 3.15 | 3.30 | 2.33 |
| MC-(PrtyrAzspe)R | 539.7 ± 114.5 | 598.8 ± 125.0 | 20.1 ± 22.9 | 26.85 | 29.79 | 6.57 | 14.82 | 2.39 |
| MC-(PrtyrAzproam)R | 156.1 ± 36.8 | 369.5 ± 4.5 | 25.5 ± 16.5 | 6.12 | 14.49 | 1.90 | 9.15 | 3.04 |
| MC-(PrtyrAzacac)R | 1031.9 ± 243.0 | 289.3 ± 90.4 | 17.3 ± 11.6 | 59.65 | 16.72 | 12.55 | 7.16 | 2.06 |
| MC-(PrtyrAzAbu)R | 145.7 ± 73.2 | 212.5 ± 54.0 | 18.0 | 8.09 | 11.81 | 1.77 | 5.26 | 2.14 |
| MC-(PrtyrAzDbu)R | 178.2 ± 104.9 | 348.1 ± 177.3 | 6.5 ± 2.8 | 27.42 | 53.55 | 2.17 | 8.62 | 0.77 |

**Tab. 3: Results from cytotoxicity assays (IC50_Cytotox) and protein phosphatase inhibition assays (IC50_PPI) for the base structure MC-(Prtyr)R (reference) and modified variants thereof. "SE" denotes standard error of at least duplicate experiments.**

| Microcystin (MC) | IC_{50_}cytotox on OATP1B1 expressing cell line + SE [nM] | IC_{50_}cytotox on OATP1B3 expressing cell line + SE [nM] | IC_{50_}PPI + SE [nM] | Ratio of IC_{50_}cytotox OATP1B1/ IC_{50_}PPI | Ratio of IC_{50_}cytotox OATP1B3/ IC_{50_}PPI | Ratio of IC_{50_}cytotox OATP1B1 (PrtyrmodR/ PrtyrR) | Ratio of IC_{50_}cytotox OATP1B3 (PrtyrmodR/ PrtyrR) | Ratio of IC_{50_}PPI (PrtyrmodR/ PrtyrR) |
|---|---|---|---|---|---|---|---|---|
| MC-PrtyrR (base structure) | 82.2± 4.5 | 40.4 ± 36.6 | 8.4 ± 5.4 | 9.79 | 4.81 | | | |
| Compound 1 | 567.6 | 187.3 | 17.5 ± 9.8 | 32.43 | 10.70 | 6.91 | 4.64 | 2.08 |
| Compound 2 | 36.4 | 128.2 | 5.0 ± 6.3 | 7.28 | 25.64 | 0.44 | 3.17 | 0.60 |

## Claims

1. A method of modifying a cyanobacterial or fungal toxin for use as a payload in an antibody-drug conjugate, comprising the following steps:
- selecting a cyanobacterial or fungal toxin having a base structure known to be cytotoxic for a target cell after uptake by a transporter protein of the target cell,
- modifying the base structure with at least one structural modification,
wherein the at least one structural modification comprises
an organic inhibitory group or effector molecule reducing the uptake of the modified toxin by the transporter protein into the target cell in comparison to the base structure, and
an organic coupling group configured for covalent conjugation of the modified toxin to an antibody.

2. The method according to claim 1, wherein the base structure comprises a cyclic oligopeptide with several amino acids and wherein modifying the base structure comprises incorporating the at least one structural modification in, or attaching the at least one structural modification to, a side-chain of at least one of the amino acids.

3. The method according to any one of claims 1 and 2, wherein the at least one structural modification comprises an organic moiety including both the organic inhibitory group and the organic coupling group.

4. The method according to claim 2, wherein the at least one structural modification comprises a first structural modification comprising the organic inhibitory group or effector molecule and a second structural modification comprising the organic coupling group, and
wherein modifying the base structure comprises incorporating the first structural modification in, or attaching the first structural modification to, a side-chain of a first amino acid, and incorporating the second structural modification in, or attaching the second structural modification to, a side-chain of a second amino acid different from the first amino acid.

5. The method according to any one of claims 1 to 4, wherein the organic inhibitory group carries at least one charge at a physiological pH-value.

6. The method according to any one of claims 1 to 5, wherein the organic inhibitory group comprises an amphoteric group carrying at least one cationic charge and at least one anionic charge at a physiological pH-value.

7. The method according to any one of claims 1 to 6, wherein the effector molecule comprises a competitive or allosteric inhibitor ligand of the transporter protein, or a functional part thereof.

8. The method according to any one of claims 1 to 7, wherein the organic coupling group is selected from the group consisting of amino group, carboxyl group, hydroxyl group, azido group, alkyne group, alkene group, thiol group, aldehyde group, keto group, tetrazine group, and any combination thereof.

9. A modified cyanobacterial or fungal toxin comprising
a base structure having a cytotoxicity for a target cell after uptake by a transporter protein of the target cell and
at least one structural modification of the base structure, wherein the at least one structural modification comprises
an organic inhibitory group or effector molecule reducing the uptake of the modified toxin by the transporter protein into the target cell in comparison to the base structure, and
an organic coupling group configured for covalent conjugation of the toxin to an antibody.

10. The modified toxin according to claim 9, wherein the base structure is selected from the group consisting of a microcystin, a nodularin or an amanitin.

11. The modified toxin according to any one of claims 9 or 10, wherein the modified toxin and the base structure are inhibitors of a protein phosphatase of the target cell, and
wherein a ratio IC50_cytotox/IC50_PPI of the modified cytotoxin is greater than a ratio IC50_cytotox/IC50_PPI of the base structure,
wherein IC50_cytotox is an IC50 value of the modified toxin or base structure, respectively, with respect to the target cell and IC50_PPI is an IC50 value of the modified toxin or base structure, respectively, with respect to the protein phosphatase.

12. A use of a modified toxin according to any one of claims 9 to 11 in manufacturing an antibody-drug conjugate comprising covalently conjugating the modified toxin to an antibody or a fragment thereof comprising an antigen binding site.

13. A method for manufacturing a modified cyanobacterial or fungal toxin according to any one of claims 9 to 11, comprising the following steps:
A) providing a toxin having a base structure known to be cytotoxic for a target cell after uptake by a transporter protein of the target cell and at least one functional group capable of covalent conjugation,
B) introducing the at least one structural modification into the toxin via covalent conjugation to the functional group of the toxin.

14. An antibody-drug conjugate comprising a modified toxin of claims 9 to 11 and an antibody or a fragment thereof comprising an antigen binding site, wherein the antibody or the fragment thereof is attached to the modified toxin via the organic coupling group.

15. A modified cyanobacterial or fungal toxin according to any one of claims 9 to 11 or an antibody-drug conjugate according to claim 14 for use in the treatment of a malignant disease.
